# EUROPEAN PATENT APPLICATION

(11) **EP 1 905 422 A1**
(43) Date of publication of application: **02.04.2008**
(21) Application number: 06291536.8
(22) Date of filing: 28.09.2006
(51) Int. Cl.: A61K 8/67, A61K 8/29, A61K 8/19

(54) **Stabilized compositons containing retinoids and metal oxide pigments**

(71) Applicant: Johnson & Johnson Consumer France SAS, 92787 Issy les Moulineaux Cedex 9 (FR)
(72) Inventor: Gohier, Annie, 27310 Trinité de Thouberville (FR); Brillouet, Anne-Sophie, 27400 Lueviers (FR); Marchelidon, Hervé, 27340 Tostes (FR); Baranger, Florence, 27100 Le Vaudreuil (FR)
(74) Representative: Metten, Karl-Heinz

(57) **Abstract**

The present invention relates to a composition including (i) at least one retinoid and (ii) at least one pigment containing metal oxide, wherein the composition further includes at least one percent, by weight, of at least one tocopherol selected from the group consisting of alpha-tocopherol, beta-tocopherol, delta-tocopherol, gamma-tocopherol, or a mixture thereof.

## Description

### BACKGROUND OF THE INVENTION

Over the past few years, cosmetic products containing retinoids have been of increasing interest and have become highly successful with consumers. Retinoids have been used for the treatment of various skin problems, in particular acne, and for combating ageing and dryness of the skin.

The experience acquired in the field of the formulation of products containing retinoids has made it clear that it is difficult to obtain products wherein these active ingredients, specifically retinol, have a significant shelf life. Indeed, these agents have a tendency to become decomposed, resulting in their inactivation.

This problem of chemical stability of retinoids has led to the development of further improved formulations, in particular emulsions containing various antioxidants and/or chelators (See, e.g., US Patent No. 5,652,263). While the solutions proposed in the state of the art have been found satisfactory for certain products, they have been found to be inadequate for products containing metal oxide colored pigments, such as iron oxide containing pigments.

The present invention relates to the discovery of the ability to stabilize a composition including a retinoid and a pigment containing metal oxide by the addition of a high amount of pure tocopherol(s).

### SUMMARY OF THE INVENTION

The present invention relates to a composition including (i) at least one retinoid and (ii) at least one pigment containing metal oxide, wherein the composition further includes at least one percent, by weight, of at least one tocopherol selected from the group consisting of alpha-tocopherol, beta-tocopherol, delta-tocopherol, gamma-tocopherol, and mixtures thereof. The present invention also relates to the use of such a composition as a cosmetic article or as a part of a cosmetic article or for the preparation of a cosmetic article to be used for the topical application to skin, hair or nails.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments can be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified.

### Definitions

As used herein, "topical application" and "topically applying" means directly laying on or spreading on the skin, hair, or nail, e.g., by use of the hands or an applicator such as a wipe.

As used herein, "cosmetically-acceptable" means that cosmetically active agents, inert ingredients, or composition which the term describes are suitable for use in contact with tissues (e.g., the skin) without undue toxicity, incompatibility, instability, irritation, allergic response, and the like, commensurate with a reasonable benefit/risk ratio.

### Metal Oxide Pigments

The compositions of the present invention include one or more pigments containing metal oxide. What is meant be a pigment is a ingredient which imparts color on the skin or its appendages. Examples of metal oxides include, but are not limited to, titanium dioxide, iron oxide (e.g. Fe₂O₃ and Fe₃O₄), and silicone dioxide. In one embodiment, the composition the pigment includes an iron oxide.

In one embodiment, the compositions of the present invention include one or more multi-layered pigments. In one embodiment, the pigment has a first layer containing mica and a second layer containing a metal oxide. Such mica may be natural mica, such as muscovite mica, or chemically synthetic mica, such as synthetic fluorphlogopite. In one embodiment, the second layer substantially surrounds the first layer. What is meant by "substantially surrounds" is that it covers at least 50% of the surface area, such as at least 90% of the surface area. In one embodiment, the pigment has a first layer containing mica, a second layer containing a first metal oxide such as titanium dioxide, and a third layer containing a second metal oxide such as silicon dioxide and/or iron oxide. In one embodiment, the second layer substantially surrounds the first layer. In a further embodiment, the third layer further substantially surrounds the second layer.

Examples of multi-layered pigments include, but are not limited to: Colorona® metallic pigments, Timiron® Interference Pigments and Splendid Colors sold by Merck KgaA (Darmstadt, Germany), such as Timiron® Silk, Timiron® Super, Timiron® Splendid, and Timiron® Starlight pigments; SunShine® pigments sold by SunChemical Corporation (Cincinnati, OH), such as SunShine® Super Bronze; and Flamenco Interference colors sold by Thornley Company (Wilmington, DE), such as Flamenco Ultra Silk®.

In one embodiment, the average particle size of the pigments is less than 500 microns, such as less than 250 microns, such as less than 125 microns.

In one embodiment, the composition includes a cosmetically-acceptable amount of the one or more pigments. The pigment(s) typically will be present in the composition in an amount from about 0.001% to about 20% by weight, in particular in an amount from about 0.01% to about 10% by weight, such as from about 0.1% to about 5%, by weight, and such as from about 1% to about 3%, by weight.

### Retinoids

The compositions of the present invention contain on or more retinoids. Examples of retinoids include, but are not limited to, retinol, retinoic acid, retinal, and salts and esters thereof, such as retinyl palmitate and retinyl acetate. In one embodiment, the composition contains retinol, such as all-trans-retinol.

In one embodiment, the composition includes a cosmetically-acceptable amount of the retinoid. The retinoid typically will be present in the composition in an amount from about 0.001% to about 1% by weight, in particular in an amount from about 0.01% to about 0.2% by weight.

### Tocopherols

The compositions of the present invention include one or more tocopherols. Examples of tocopherols include, but are not limited to, alpha-tocopherol, beta-tocopherol, delta-tocopherol, and gamma-tocopherol. In one embodiment, the composition includes both alpha-tocopherol and gamma-tocopherol. Examples of such blends include Coviox® tocopherol blends, available from Cognis (Düsseldorf, Germany), which include blends of alpha-, beta, gamma-, and/or delta-tocopherols.

In one embodiment, the composition includes a cosmetically-acceptable amount of the one or more tocopherols. In one embodiment, the composition includes at least about 1%, by weight, of said at least one tocopherol. In one embodiment, the composition includes at least about 2%, by weight, of said at least one tocopherol. The tocopherol(s) typically will be present in the composition in an amount from about 1% to about 10% by weight, such as from about 2% to about 5%, by weight.

### Compositions

The compositions useful in the present invention involve formulations suitable for administering to the target tissues, such as mammalian skin such as human skin. In one embodiment, the composition contains a cosmetically-acceptable amount of (i) retinoid(s), (ii) pigment(s) containing metal oxide, (iii) tocopherol(s), and (iv) a cosmetically-acceptable carrier. In one embodiment, the cosmetically-acceptable carrier is from about 50% to about 98%, by weight, of the composition (e.g., from about 80% to about 95%, by weight, of the composition).

The compositions may be made into a wide variety of cosmetic articles that include but are not limited to lotions, creams, gels, sticks, sprays, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks, make-up such as foundations, eye liners, and eye shadows, and the like. These product types may contain several types of cosmetically- acceptable carriers including, but not limited to solutions, suspensions, emulsions such as microemulsions and nanoemulsions, gels, solids and liposomes. The following are non-limitative examples of such carriers. Other carriers can be formulated by those of ordinary skill in the art.

The compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include: propylene glycol, polyethylene glycol (e.g. 200-600), polypropylene glycol (e.g. 425-2025), glycerol, 1,2,4-butanetriol, sorbitol esters, 1,2,6-hexanetriol, ethanol, and mixtures thereof.

A lotion can be made from such a solution. Lotions typically contain from about 1% to about 20% (e.g., from about 5% to about 10%) of an emollient(s) and from about 50% to about 90% (e.g., from about 60% to about 80%) of water. As used herein, "emollients" refer to materials used for the prevention or relief of dryness, as well as for the protection of the skin or hair. Examples of emollients include, but are not limited to, those set forth in the International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "ICI Handbook").

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may contain a simple base of animal, vegetable, or synthetic oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 1693-1697.

The compositions useful in the present invention can also be formulated as emulsions. If the carrier is an emulsion, regularly from about 1% to about 10% (e.g., from about 2% to about 5%) of the carrier contains an emulsifier(s). Emulsifiers may be nonionic, anionic or cationic. Examples of emulsifiers include, but are not limited to, those set forth in the ICI Handbook, pp.1673-1686.

Lotions and creams can be formulated as emulsions. Typically such lotions contain from 0.5% to about 5% of an emulsifier(s), while such creams would typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single phase emulsion skin care preparations, such as lotions and creams, of the oil-in-water type and water-in-oil type are well-known in the art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contain between about 0.1% and 5%, by weight, of such gelling agents.

The compositions of the present invention can also be formulated into a solid formulation (e.g., a wax-based stick, soap bar composition, powder, and wipe containing powder).

The compositions useful in the subject invention may contain, in addition to the aforementioned components, a wide variety of additional oil-soluble materials and/or water-soluble materials conventionally used in compositions for use on skin at their art-established levels.

### Additional Cosmetically Active Agents

In one embodiment, the topical composition further includes cosmetically active agent. What is meant by a "cosmetically active agent" is a compound that has a cosmetic or therapeutic effect on the skin, hair, or nails, e.g., lightening agents, darkening agents such as self-tanning agents, anti-acne agents, shine control agents, anti-microbial agents, anti-inflammatory agents, antimycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, and/or skin conditioning.

In one embodiment, the cosmetically active agent is selected from, but not limited to, the group consisting of hydroxy acids, benzoyl peroxide, sulfur resorcinol, ascorbic acid, D-panthenol, hydroquinone, octyl methoxycinnimate, titanium dioxide, octyl salicylate, homosalate, avobenzone, polyphenolics, carotenoids, free radical scavengers, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, coenzyme Q10, lipoic acid, amino acids such a proline and tyrosine, vitamins, lactobionic acid, acetyl-coenzyme A, niacin, riboflavin, thiamin, ribose, electron transporters such as NADH and FADH2, and other botanical extracts such as aloe vera, feverfew, and soy, and derivatives and mixtures thereof. The cosmetically active agent will typically be present in the composition of the invention in an amount of from about 0.001% to about 20% by weight of the composition, e.g., about 0.01% to about 10% such as about 0.1% to about 5%.

Examples of vitamins include, but are not limited to, vitamin Bs (such as vitamin B3, vitamin B5, and vitamin B12), vitamin C, and vitamin K, and derivatives thereof.

In one embodiment, the composition also contains an antioxidant. Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, ascorbic acid, and ascorbic acid derivatives (e.g., ascorbyl palmitate and ascorbyl polypeptide). Oil-soluble antioxidants suitable for use in the compositions of this invention include, but are not limited to, butylated hydroxytoluene, tocopherol derivatices (e.g., tocopheryl acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis. Other examples of antioxidants may be found on pages 1612-13 of the ICI Handbook.

### Other Materials

Various other cosmetically-active agents may also be present in the skin care products. These include, but are not limited to, skin protectants, humectants, and emollients. The composition may also include chelating agents (e.g., EDTA), preservatives (e.g., parabens), pigments, dyes, opacifiers (e.g., titanium dioxide), and fragrances.

### Uses

The composition according to the invention can be used to impart color to the skin, hair, or nails (e.g., as make-up or to impart the appearance of a tan on the skin) as well as to treat a variety of skin conditions, such as the signs of aging (e.g., reducing the appearance of wrinkles and fine lines), acne, light or dark areas of the skin, visible pores and oil on the skin, and dry skin.

The composition and products containing such compositions of the present invention may be prepared using methodology that is well known by an artisan of ordinary skill.

### Example 1

The composition of Table 1 was manufactured as follows. Ingredients H, I, J, and K were mixed and homogenized at room temperature ("Premix 1"). Separately, ingredients L and M were mixed with about 5% percent of the water of ingredient A at room temperature ("Premix 2"). Separately, ingredients N, O, P, Q, and R were mixed and homogenized at room temperature ("Premix 3"). Separately, the remainder of ingredient A and Ingredients B, C, D, E, and F were mixed and heated to 60°C, following which ingredient G was added to the resulting mixture and further mixed, following which Premix 1 was further added to the resulting mixture and further mixed.

The resulting mixture was allowed to cool to about 45°C, at which time Premix 2 was added and mixed. The resulting mixture was then allowed to further cool to about 35°C, at which time Premix 3 was added and mixed. The resulting mixture was further allowed to cool to about 30°C, at which time ingredients S, T, U, V, W, and X were added and mixed. The resulting composition had a pH of about 7 and a viscosity of about 6000mPas with a physica apparatus.

**Table 1**

| | INGREDIENT INCI NAME | %(W/W) |
|---|---|---|
| A | Water | QS 100% |
| B | Ammonium Acryloydimethyltaurate/VP copolymer and Water (Aristoflex AVC, Clariant GMbH, Frankfort, Germany) | 0.1-3% |
| C | Glycerin | 0.1-10% |
| D | Disodium EDTA | 0.01% -0.10% |
| E | Phenoxyethanol | 0.001-1% |
| F | Methylparaben | 0.001-0.5% |
| G | C13-14 Isoparaffin, laureth G 7, Polyacrylamide, and water (Sepigel 305, Seppic, Castres, France) | 0.1-2% |
| H | Alumina, aluminium stearate, water, diazolidinyl urea,isodeceth-6,methylparaben,oleth-10,propylene glycol, propylparaben, simethicone, titanium dioxide (SOLAVEIL, Uniquema, Gouda, Netherlands) | 2-15% |
| I | PEG-12 Dimethicone, CI77491 (AQUACHROME RED, Trendcosmetic, Inc., New Jersey, USA) | 0.001-1% |
| J | PEG-12 Dimethicone, CI77492 (AQUACHROME YELLOW, Trendcosmetic, Inc) | 0.001-1% |
| K | PEG-12 Dimethicone, CI77499 (AQUACHROME BLACK, Trendcosmetic, Inc) | 0.001-1% |
| L | TETRAHYDROXYPROPYL Ethylenediamine | 0.1-80 0.1-8% |
| M | citric acid | 0.001-1% |
| N | Cyclopentasiloxane | 0.1-3% |
| O | BHT | 0.01%-0.1% |
| P | tocopherol, Helianthus Annuus (Coviox T90EU, Cognis, Düsseldorf, Germany) | 0.1-3% |
| Q | Bisabolol | 0.1-0.5% |
| R | PEG-12 Dimethicone | 0.1-3% |
| S | Laureth-4, laureth - 23,Dimethicone, dimethicone Crosspolymer, water, and cyclopentasiloxane (DC 7-3118, Dow Corning, Seneffe, Belgium) | 1-35% |
| T | Ascorbic Acid | 0.01-0.5% |
| U | BHT, Polysorbate 20,and Retinol (RETINOL 50C, Roche, BASF, Ludwigshafen, Germany) | 0.01-0.3% |
| V | Mica, Silica, and CI77891 (Timiron Splendid Gold) | 0.1-3% |
| W | CI77491, Synthetic Fluorphlogopite (SunShine Super Bronze) | 0.1-3% |
| X | Mica, CI 77891 (Flamenco Ultra Silk) | 0.1-3% |

### Example 2

The following seven examples of Table 2 were made according the process of Example 1, wherein the type of pigments and amount of tocopherols where varied.

**Table 2**

| INGREDIENT INCI NAME | 1 | 2 | 3 | 4 | 5 | 6 | 7 |
|---|---|---|---|---|---|---|---|
| tocopherol, Helianthus Annuus | 0% | 0.1% | 1% | 2% | 0% | 1% | 2% |
| Mica, Silica (timiron super red and/or timiron super violet) | 0 | 0 | 0 | 0 | 1% | 1% | 1% |
| CI77491, Synthetic Fluorphlogopite (SunShine Super Bronze) | 1% | 1% | 1% | 1% | 0% | 0% | 0% |

The above seven formulations were tested in an accelerated stability assay to determine the stability of retinol in the formulations. During the assay, the formulations were exposed to steady oxygen flow for 72 hrs at room temperature.

The results of the assay are depicted in Table 3.

**Table 3**

| Composition No. | Percent Retinol Degradation |
|---|---|
| 1 | 44% |
| 2 | 22% |
| 3 | 10% |
| 4 | 7% |
| 5 | 31% |
| 6 | 10% |
| 7 | 7% |

Thus, the addition of tocopherols unexpectedly and significantly enhanced the stability of retinol in the presence of metal oxide containing pigments.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A composition comprising (i) at least one retinoid and (ii) at least one pigment comprising metal oxide, wherein said composition further comprises at least one percent, by weight, of at least one tocopherol selected from the group consisting of alpha-tocopherol, beta-tocopherol, delta-tocopherol, gamma-tocopherol, or a mixture thereof.

2. A composition according to claim 1, wherein at least one of said retinoid is retinol.

3. A composition according to claim 1 or 2, wherein at least one of said tocopherol comprises gamma-tocopherol.

4. A composition according to one of the preceding claims, wherein said pigment comprises iron oxide.

5. A composition according to one of the preceding claims, wherein at least one of said pigment is a multi-layered pigment, wherein said pigment comprises a first layer comprising mica and a second layer comprising metal oxide.

6. A composition according to claim 5, wherein said second layer substantially surrounds said first layer.

7. A composition according to one of the preceding claims, wherein at least one of said pigment is a multi-layered pigment, wherein said pigment comprises a first layer comprising mica, a second layer that substantially surrounds said first layer, and a third layer that substantially surrounds said second layer.

8. A composition according to claim 7, wherein said second layer comprises titanium dioxide and said third layer comprises at least one metal oxide selected from iron oxide and silicon dioxide.

9. A composition according to one of the preceding claims, wherein the average particle size of said at least one pigment, in particular multi-layered pigment, comprised within said composition is less than 500 microns.

10. A composition according to one of the preceding claims, wherein said composition comprises at least 0.2%, by weight, of said at least one pigment.

11. A composition according to one of the preceding claims, wherein said composition is in the form of an emulsion.

12. Use of a composition according to anyone of the preceding claims as a cosmetic article or as a part of a cosmetic article or for the preparation of a cosmetic article to be used for the topical application to skin, hair or nails.
